# EUROPEAN PATENT APPLICATION

(11) **EP 4 628 084 A1**
(43) Date of publication of application: **08.10.2025**
(21) Application number: 23897718.5
(22) Date of filing: 27.11.2023
(51) Int. Cl.: A61K 31/655, A61F 9/007, A61K 9/19, A61K 47/04, A61P 27/02

(54) **NAPHTHALENE-DERIVATIVE-BASED THERAPEUTIC DRUG FOR OPHTHALMOLOGICAL DISEASE**

(30) Priority: 28.11.2022 JP 2022189103
(71) Applicant: Kyoto University, Kyoto-shi, Kyoto 606-8501 (JP); Kyoto Drug Discovery & Development Co., Ltd., Ibaraki-shi, Osaka, 567-0085 (JP)
(72) Inventor: KAKIZUKA, Akira, Kyoto-shi, Kyoto 606-8501 (JP); IKEDA, Hanako, Kyoto-shi, Kyoto 606-8501 (JP); MUSASHI, Kunihiro, Ibaraki-shi, Osaka 567-0085 (JP); OKAMOTO, Masaki, Ibaraki-shi, Osaka 567-0085 (JP)
(74) Representative: Hoffmann Eitle
(86) International application number: PCT/JP2023/042321
(87) International publication number: WO 2024/117070

(57) **Abstract**

The present invention relates to a drug product comprising KUS121 that can be administered into the vitreous body.

## Description

### TECHNICAL FIELD

The present invention relates to a drug product comprising KUS121 that can be administered into the vitreous body.

### BACKGROUND OF THE INVENTION

Sodium 4-amino-3-[6-(4-fluoro-2-methylphenyl)pyridin-3-ylazo]naphthalene-1-sulfonate dihydrate (hereinafter, also referred to as "KUS121") is a compound having a naphthalene structure, which has been now developed as a medicament for treating retinal ischemic disease and retinal degenerative disease (Patent literature 1). One of the recommended administration methods for KUS121 is as an injection that is directly injected into the vitreous body of the eye.

When KUS121 is injected into the vitreous body, phosphate buffered saline (PBS buffer) is considered to be very suitable as an administration vehicle, taking into account the physical properties of the intravitreal fluid, such as pH (about 7.5) and osmolarity (about 290 mOsm/kg), as well as the past safety record of intravitreal injection.

On the other hand, the volume of the vitreous body is limited to about 4 mL, and injecting a large volume of the administered solution into the vitreous body may damage the intraocular tissues and increase intraocular pressure. Thus, the administration volume needs to be as small as possible (about 100 µL or less), and the required dose of the drug needs to be contained in this small volume of the administration solution.

In preparing a KUS121-containing administration solution, the solubility of KUS121 in water (at 25°C) is relatively high at about 2.8 mg/mL, whereas the solubility of KUS121 in PBS buffer is extremely low due to the ionic effect of the high concentration of inorganic ions in the buffer. In order to dissolve a given amount of KUS121 in PBS buffer and administer it into the vitreous body, it is necessary to significantly increase the amount of PBS buffer relative to the amount of KUS121. However, as mentioned above, when administering into the vitreous body, it is necessary to minimize the amount of an injection solution, and it is inappropriate to increase the amount of PBS buffer.

In addition, since the osmotic pressure of a normal PBS buffer is about the same as the osmotic pressure in the vitreous body (about 290 mOsm/kg), it was inappropriate to reduce the concentration of the PBS buffer considering the extremely low solubility of KUS121 in a PBS buffer as described above, as this dilution would lower the osmotic pressure.

Even if trying to increase the solubility by adding a dissolution aid as another means of improving solubility, it is extremely difficult to select an appropriate dissolution aid because the site of administration is the vitreous body, which is a delicate area. Furthermore, even if KUS121 is dissolved in PBS buffer by warming prior to administration, it is not practical to perform a series of operations in an actual medical setting, such as quickly dissolving it by warming, cooling it to an appropriate temperature, and then injecting it into the vitreous body of the eye. Alternatively, even if a dissolution solution other than PBS buffer is to be considered, it would not be easy to consider such another solution, considering the osmotic pressure matched to the intravitreal fluid, safety, etc.

### PRIOR ART

### (Patent Reference)

[Patent literature 1] WO 2012/014994

### SUMMARY OF INVENTION

### (Technical Problem)

The purpose of the present invention is to prepare an injection formulation comprising KUS121 that is directly injected into the vitreous body, in which KUS121 is dissolved in a PBS buffer solution and adjusted to a predetermined concentration, the dissolved state can be maintained for a certain period, and the pH and osmotic pressure of the administration solution are adjusted to the same level as in the vitreous body.

### (Solution to Problem)

The maximum dose of KUS121 to the vitreous body of the eye is 50 µg/eye, and the liquid volume that can be administered into the vitreous body is preferably about 100 µL or less. Further, in a preliminary experiment, it was found that the soluble concentration of KUS121 in a normal PBS buffer solution is 0.01 mg/mL or less at room temperature. In view of these premises and the above-mentioned problems, the present inventors have extensively studied to design an injection formulation of KUS121 in a normal PBS buffer that can be administered into the vitreous body, wherein about 50 µg of KUS121 is dissolved in a normal PBS buffer (about 100 µL), *i.e.,* a formulation that allows a concentration setting of 0.5 mg/mL and matches the osmotic pressure in the vitreous body. As a result, the present inventors have conducted the following trials and then have found the following things: *i.e.,* the present inventors first prepared 1 mL of 0.1 mg/mL KUS121 aqueous solution by dissolving KUS121 in water for injection and then adding a PBS buffer solution with about twice the concentration of normal to the KUS121 solution, in which the PBS concentration is 1/5 of the normal concentration. Then, the prepared KUS121 aqueous solution was lyophilized, and 200 µL (0.2 mL) of water for injection [200 µL (0.2 mL) corresponds to 1/5 the volume before the lyophilization] was added to the lyophilized product. As a result, the lyophilized formulation comprising KUS121 was completely dissolved even though the drug concentration was 5 times higher than the concentration before the lyophilization and the drug concentration was more than 50 times higher than its solubility. Furthermore, it has been also found that the dissolved state remained stable for more than 24 hours. Based upon the new findings, the present invention has been completed. One of the administration solutions of the present invention is an administration solution in which 50 µg of KUS121 is dissolved in 100 µL of PBS buffer at the maximum dose, and is adjusted to the pH and osmotic pressure in the vitreous body.

The present invention includes the following embodiments.

(Item 1) A lyophilized drug product prepared by (i) dissolving sodium 4-amino-3-[6-(4-fluoro-2-methylphenyl)pyridin-3-ylazo]naphthalene-1-sulfonate dihydrate (hereinafter, abbreviated as "KUS121") in water for injection or a PBS buffer solution more dilute than usual, (ii) adding a PBS buffer thicker than usual to the solution while maintaining the dissolved state of the solution, and (iii) lyophilizing the obtained solution,
   which is characterized in that, when the lyophilized product is dissolved in water for injection prior to administration, the solution remains in solution state, in which the concentration of KUS121 is 0.100 to 0.625 mg/mL (preferably, 0.125 to 0.5 mg/mL), and the concentration of PBS is the normal PBS concentration.
(Item 2) The lyophilized drug product of Item 1, wherein the PBS buffer thicker than usual has a concentration 2 to 10 times higher than the normal PBS concentration.
(Item 3) The lyophilized drug product of Item 1 or 2, wherein the PBS concentration of the solution before (iii) lyophilization step is 1/5 to 1/1 (preferably 1/5 to 1/2) of the normal PBS concentration.
(Item 4) The lyophilized drug product of any one of Items 1 to 3, wherein the volume of the water for injection added to the lyophilized product is 1/5 to 1/1 (preferably, 1/5 to 1/2) of the volume of the solution before (iii) lyophilization step.
(Item 5) The lyophilized drug product of any one of Items 1 to 4, wherein the osmotic pressure of the solution prepared by dissolving the lyophilized product in water for injection is 230 to 360 mOsm/kg.
(Item 6) The lyophilized drug product of any one of Items 1 to 5, wherein the pH of the solution prepared by dissolving the lyophilized product in water for injection is 6.8 to 8.2.
(Item 7) The lyophilized drug product of any one of Items 1 to 4, wherein the normal PBS concentration is a concentration of PBS buffer with an osmolarity of 230 to 360 mOsm/kg and a pH of 6.8 to 8.2.
(Item 8) A lyophilized drug product prepared by (i) dissolving KUS121 in water for injection, (ii) adding a PBS buffer with a concentration 2 to 10 times (preferably about two times) higher than the normal PBS concentration to the KUS121 solution, in which the PBS concentration is 1/5 to 1/2 (preferably about 1/5) of the normal concentration, while maintaining the dissolved state of the solution, and (iii) lyophilizing the obtained solution,
   which is characterized in that, when the lyophilized product is dissolved in water for injection in a volume of 1/5 to 1/2 (preferably about 1/5) of the solution before (iii) lyophilization step prior to administration, the solution remains in solution state, in which the concentration of KUS121 is 0.100 to 0.625 mg/mL (preferably, 0.125 to 0.5 mg/mL), and the concentration of PBS is the normal PBS concentration.
(Item 9) The lyophilized drug product of any one of Items 1 to 8, wherein the KUS solution comprising a PBS buffer before (iii) lyophilization step is dispensed into each vial in which the amount of KUS121 is 20 to 125 µg (preferably, 25 to 100 µg) per vial, said drug product is stored in the vial in lyophilized state.
(Item 10) A vial containing a lyophilized drug product comprising KUS121 which is for vitreous injection, wherein the lyophilized drug product comprises 20 to 125 µg (preferably, 25 - 100 µg) of KUS121 and PBS buffer dry components corresponding to 0.1 to 0.4 mL (preferably 0.2 mL) of a normal PBS buffer solution, excluding water.
(Item 11) A method for preparing a lyophilized drug product that can be dissolved in water for injection to prepare a PBS buffer solution comprising 0.100 to 0.625 mg/mL (preferably, 0.125 to 0.5 mg/mL) of KUS121, wherein the PBS concentration is the normal PBS concentration, comprising
   dissolving KUS121 in water for injection or a PBS buffer solution more dilute than usual,
   adding a PBS buffer thicker than usual to the solution while maintaining the dissolved state of the solution, and
   lyophilizing the obtained solution.
(Item 12) The method of Item 11, wherein the normal PBS concentration is a concentration of a PBS buffer with an osmotic pressure of 230 to 360 mOsm/kg, and a pH of 6.8 to 8.2.
(Item 13)
   The method of Item 11 or 12, wherein the PBS buffer thicker than usual has a concentration 2 to 10 times higher than the normal PBS concentration.
(Item 14) The method of any one of Items 11 to 13, wherein the PBS concentration of the solution before (iii) lyophilization step is 1/5 to 1/1 (preferably 1/5 to 1/2) of the normal PBS concentration.
(Item 15) The method of any one of Items 11 to 14, wherein the volume of the water for injection added to the lyophilized product is 1/5 to 1/1 (preferably, 1/5 to 1/2) of the volume of the solution before (iii) lyophilization step.
(Item 16) The method of any one of Items 11 to 14, wherein the PBS buffer solution comprising KUS121 can be prepared by dissolving the lyophilized drug product in 200 µL of water for injection per vial.
(Item 17) The method of any one of Items 11 to 16, wherein the concentration of KUS121 in the solution before the lyophilization step is 0.020 to 0.125 mg/mL (preferably, 0.025 to 0.1 mg/mL).
(Item 18) The lyophilized drug product of any one of Items 1 to 9, the vial of Item 10, or the method of any one of Items 11 to 17, wherein [Na⁺], [K⁺], [Cl⁻], and [PO₄³⁻] in a solution obtained by dissolving the lyophilized drug product in water for injection are 100 to 200 mmol/L, 0 to 6.5 mmol/L, 90 to 180 mmol/L, and 6 to 14 mmol/L, respectively.

### (Effect of the Invention)

In the present invention, once KUS121 is completely dissolved in water for injection which has high solubility for KUS121, and a PBS buffer solution with a higher concentration than usual is added to the solution while maintaining the dissolved state to prepare a PBS solution with a concentration about 5 times dilute than usual. Thereby, the dissolved state of the above-obtained solution is more stable than that obtained by dissolving KUS121 in a supersaturated state in a PBS buffer solution with a normal concentration, and there is almost no problem of precipitation of KUS121 during operations such as sterile filtration before lyophilization.

In the present invention, a solution obtained by dissolving the lyophilized drug product in water for injection can be smoothly prepared even though KUS121 at the same concentration does not dissolve in a PBS buffer, and its dissolved state is very stable and suitable as an administration preparation of the present invention even though it is considered to be in a supersaturated state.

### DESCRIPTION OF EMBODIMENTS

The active ingredient of the present invention, KUS121, is sodium 4-amino-3-[6-(4-fluoro-2-methylphenyl)pyridin-3-ylazo]naphthalene-1-sulfonate dihydrate (hereinafter, abbreviated as "KUS121"). In the drug product of the present invention, however, KUS121 is once dissolved in a PBS buffer solution and then lyophilized, thereby it does not necessarily exist as a dihydrate or sodium salt in the drug product. Thus, it can be the anhydrous form, as well as salts with other cations such as potassium, calcium, and magnesium salts, and in the salt-free state, hence the definition of KUS121 includes these analogs.

Ordinarily, KUS121 exists stably as a dihydrate, but in the present invention, the concentration thereof is calculated on an anhydrous basis.

In the present invention, the "PBS buffer solution thicker than usual" means that it is thicker than the normal PBS concentration, and in particular, there is no limitation as long as when it is added to the KUS121 solution that is once dissolved before lyophilization, the dissolved state thereof is maintained and the PBS concentration in the solution that is prepared by adding water for injection after lyophilization can be adjusted to the normal PBS concentration. The "PBS buffer solution thicker than usual" is, for example, 2 to 10 times the normal PBS concentration, preferably 2 to 5 times, more preferably 2 to 3 times, still more preferably about 2 times.

In the present invention, the "PBS buffer solution more dilute than usual" is not particularly limited as long as it is more dilute than the usual PBS concentration. Since the "PBS buffer more dilute than usual" is written in parallel with "water for injection" like "water for injection or a PBS buffer more dilute than usual", the lower limit of "PBS buffer more dilute than usual" is intended to be water for injection, that is, the PBS concentration is 0. With respect to "water for injection or a PBS buffer solution more dilute than usual", water for injection is preferably used. Moreover, the water for injection here may be purified water, considering sterile filtration before lyophilization.

The state in which KUS121 is dissolved before lyophilization does not necessarily mean that it is dissolved in a normal state, but includes a case in which it is dissolved in a supersaturated state.

The "volume of the water for injection added to the lyophilized product" is not particularly limited as long as the PBS concentration in the solution dissolved by adding water for injection becomes the normal PBS concentration. For example, if the PBS concentration of the solution before lyophilization is 1/5 of the normal concentration; by adding water for injection in a volume of 1/5 of the volume of the solution before lyophilization, the PBS concentration of the administration solution will become the normal PBS concentration. The "volume of the water for injection added to the lyophilized product" is, for example, 1/5 to 1/1, preferably 1/5 to 1/2, more preferably about 1/5.

In the present invention, the amount of KUS121 administered into the vitreous body is 12.5 µg/eye, 25 µg/eye, or 50 µg/eye, or 12.5 µg to 50 µg/eye in one administration. Alternatively, these dosages may be reduced as appropriate depending on the patient's condition.

In the drug product of the present invention, for example, a lyophilized preparation comprising 0.025 to 0.1 mg of KUS121 is dissolved in 200 µL of water for injection, and 100 µL of the solution is injected into the vitreous body, thereby making it possible to administer KUS121 in an amount of 12.5 to 50 µg/eye.

The pH of the solution when dissolved in water for injection is 6.8 to 8.2, preferably 7.0 to 8.0, more preferably 7.2 to 7.8.

The osmotic pressure of the solution when dissolved in water for injection is 230 to 360 mOsm/kg, preferably 250 to 330 mOsm/kg.

In the present invention, "phosphate buffered saline (PBS buffer)" is not particularly limited as long as it is a PBS buffer with a commonly prepared composition or a commercially available PBS buffer, and it comprises Na⁺, K⁺, H⁺, Cl⁻, PO₄³⁻, OH⁻ ions, some do not comprise K⁺, some comprise Ca²⁺ and Mg²⁺, and the pH is adjusted to 7.2 to 7.4. An example of a common PBS buffer has the composition shown in the table below. And, some materials have multiple different hydrates, but the table below shows the amount used in terms of anhydride, and the type of hydrate does not matter.

| Material | Amount |
|---|---|
| sodium chloride | 8.00 g |
| disodium hydrogen phosphate | 1.15 g |
| potassium dihydrogen phosphate | 0.20 g |
| potassium chloride | 0.20 g |
| water for injection | Total amount of all ingredients is 1000.00 g, or all ingredients are dissolved to 1 L. |

In the present invention, "PBS concentration" is an index using the concentration of the above-mentioned normal PBS buffer as a standard. For example, if it is 1/5 of the normal concentration, it means that the above-mentioned ion concentrations are diluted by 1/5, and if it is 2 times, it means that the above-mentioned ion concentrations are concentrated by 2 times.

In the present invention, the term "normal PBS concentration" refers to a concentration in a commonly prepared PBS buffer or a commercially available PBS buffer, and includes, for example, a PBS buffer having the composition shown in the table above. Particularly, in the present invention, the purpose of setting the PBS concentration of the administration solution as a "normal PBS concentration" is to set a PBS concentration that matches pH (about 7.5) and osmotic pressure (about 290 mOsm/kg) in the vitreous body, and as long as the specific pH and osmotic pressure shown in the above paragraph are satisfied, the range of the "normal PBS concentration" in the present invention is up to a concentration including a certain variation in the normal PBS concentration.

The method for preparing the drug product of the present invention is to (i) dissolve KUS121 completely in water for injection or a PBS buffer solution more dilute than usual, (ii) add a PBS buffer thicker than usual to the solution while maintaining the dissolved state of the solution, (iii) filter the solution aseptically, and (iv) lyophilize the filtrate. The method has no particular limitations as long as a PBS buffer solution of 0.100 to 0.625 mg/mL (preferably, 0.125 to 0.5 mg/mL) of KUS121 can be prepared by dissolving the lyophilized preparation in water for injection, and the PBS concentration of the dissolved solution is around the normal PBS concentration. In this case, the osmotic pressure is comparable to that of a normal PBS solution, that is, the osmotic pressure in the vitreous body.

### EXAMPLES

### Example 1

To a stainless steel cup were added 16.00 g of sodium chloride, 2.29 g (anhydrous basis) of disodium hydrogen phosphate, 0.400 g of potassium dihydrogen phosphate, and 0.400 g of potassium chloride. The mixture was dissolved in water for injection, and the total amount thereof was made up to 1000 g to prepare a PBS buffer solution with twice the concentration of normal.

To another stainless steel cup was added 0.0486 g (anhydrous basis: 0.0450 g) of KUS121, and it was dissolved in 405 mL of water for injection. To the solution was slowly added 45 mL of the above-prepared 2x PBS buffer with mixing to prepare the final KUS121 preparation for lyophilization.

The final KUS121 preparation was sterile filtered, but about 100 mL of the initial stream was discarded. After filling 1 mL of the sterile filtrate into a vial and semi-sealing it with a rubber stopper, the vial was transferred to a lyophilizer and lyophilized to obtain a lyophilized preparation.

### Example 2

To a 5 L beaker were added 1600 g of water for injection, 32.06 g of sodium chloride, 4.59 g (anhydrous basis) of disodium hydrogen phosphate, 0.80 g of potassium dihydrogen phosphate, and 0.80 g of potassium phosphate. The mixture was dissolved in water for injection, and the total amount thereof was made up to 2000 g to prepare a PBS buffer solution with twice the concentration of normal.

To a 5 L glass vessel was added 0.484 g (anhydrous basis: 0.449 g) of KUS121, and it was dissolved in water for injection, and the total amount thereof was made up to 2250 g.

The above-prepared KUS121 solution was transferred to a 10 L disposable bag, and mixed with 877 g of the above-prepared 2x PBS buffer and an appropriate amount of water for injection. Further, water for injection was added thereto to make the total amount 8770 g, which was used as the final KUS121 preparation for lyophilization.

The final KUS121 preparation was sterile filtered, but about 700 mL of the initial stream was discarded. After filling 1 mL of the sterile filtrate into a vial and semi-sealing it with a rubber stopper, the vial was transferred to a lyophilizer and lyophilized to obtain a lyophilized preparation.

### Example 3 (Dissolution of lyophilized preparation)

Into the vial containing the above-prepared lyophilized preparation comprising KUS121 (0.1 mg), sodium chloride (1.6 mg), disodium hydrogen phosphate (0.229 mg), potassium dihydrogen phosphate (0.04 mg), and potassium chloride (0.04 mg) per vial, 200 µL of water for injection was added using a needle-equipped syringe. The mixture was mixed and the time until dissolution was measured with a stopwatch. The dissolution time was less than 90 seconds.

### Example 4 (Stability of dissolved liquid preparation)

Into the vial containing the above-prepared lyophilized preparation comprising KUS121 (0.1 mg or 0.05 mg), sodium chloride (1.6 mg), disodium hydrogen phosphate (0.229 mg), potassium dihydrogen phosphate (0.04 mg), and potassium chloride (0.04 mg) per vial, 200 µL of water for injection was added using a needle-equipped syringe, and the mixture was dissolved to prepare a PBS buffer solution of KUS121 (0.5 mg/mL or 0.25 mg/mL, the PBS concentration is 1/1). The appearance of the solution at room temperature, the contents of KUS121 and related substances in the solution, the presence of insoluble foreign substances, and the color of the solution were measured at the time of dissolution, after 3 hours, after 6 hours, after 12 hours, and after 24 hours. In both the two types of the dissolved preparations with different concentrations of KUS121, the dissolved state was maintained until 24 hours later, and no change was observed in any of the measurements.

On the other hand, a solution having the same composition as above without via lyophilization was prepared as follows. KUS121 (0.5 mg) was dissolved in water for injection (0.9 mL), and a PBS buffer (0.1 mL) at 10 times the normal concentration, which comprises sodium chloride (8.00 mg), disodium hydrogen phosphate (1.15 mg), potassium dihydrogen phosphate (0.2 mg), and potassium chloride (0.2 mg) per 0.1 mL, was slowly added to the KUS121 solution to prepare a PBS solution (0.5 mg/mL, PBS concentration 1/1) of KUS121 maintained in dissolved state. When this solution was stored at room temperature, a solid of KUS121 was precipitated in 3 hours.

Comparing the appearances of the two solutions with the same composition at 0.5 mg/mL as described above, the solution via lyophilization maintained a dissolved state for 24 hours, whereas the solution without via lyophilization resulted in solid precipitation in 3 hours.

### Example 5 (Solubility of KUS121 in PBS buffer)

A PBS buffer solution comprising 1.6 mg of sodium chloride, 0.229 mg of disodium hydrogen phosphate, 0.04 mg of potassium dihydrogen phosphate, and 0.04 mg of potassium chloride per mL of the solution was prepared, and the temperature of the PBS buffer solution was adjusted to 5°C, 25°C, 40°C, 50°C, and 60°C. KUS121 was added little by little to each solution at the different temperatures and stirred. After stirring for a sufficient time period from the time when some undissolved material appeared, the solution was filtered. As a result of quantifying the filtrate using HPLC, the solubility of KUS121 at each temperature was as follows (detection limit: about 1 µg/mL, quantification limit: 5 µg/mL) . 5°C: below detection limit, 25°C: below detection limit, 40°C: 7 µg/mL, 50°C: 47 µg/mL, 60°C: 132 µg/mL.

## Claims

1. A lyophilized drug product prepared by (i) dissolving sodium 4-amino-3-[6-(4-fluoro-2-methylphenyl)pyridin-3-ylazo]naphthalene-1-sulfonate dihydrate (hereinafter, abbreviated as "KUS121") in water for injection or a PBS buffer solution more dilute than usual, (ii) adding a PBS buffer thicker than usual to the solution while maintaining the dissolved state of the solution, and (iii) lyophilizing the obtained solution,
which is **characterized in that**, when the lyophilized product is dissolved in water for injection prior to administration, the solution remains in solution state, in which the concentration of KUS121 is 0.100 to 0.625 mg/mL, and the concentration of PBS is the normal PBS concentration.

2. The lyophilized drug product of claim 1, wherein the PBS buffer thicker than usual has a concentration 2 to 10 times higher than the normal PBS concentration.

3. The lyophilized drug product of claim 1 or 2, wherein the PBS concentration of the solution before (iii) lyophilization step is 1/5 to 1/1 (preferably 1/5 to 1/2) of the normal PBS concentration.

4. The lyophilized drug product of any one of claims 1 to **3,** wherein the volume of the water for injection added to the lyophilized product is 1/5 to 1/1 (preferably, 1/5 to 1/2) of the volume of the solution before (iii) lyophilization step.

5. The lyophilized drug product of any one of claims 1 to **4,** wherein the osmotic pressure of the solution prepared by dissolving the lyophilized product in water for injection is 230 to 360 mOsm/kg.

6. The lyophilized drug product of any one of claims 1 to **5,** wherein the pH of the solution prepared by dissolving the lyophilized product in water for injection is 6.8 to 8.2.

7. The lyophilized drug product of any one of claims 1 to **4,** wherein the normal PBS concentration is a concentration of PBS buffer with an osmolarity of 230 to 360 mOsm/kg and a pH **of 6.8 to 8.2.**

8. A lyophilized drug product prepared by (i) dissolving KUS121 in water for injection, (ii) adding a PBS buffer with a concentration 2 to 10 times higher than the normal PBS concentration to the KUS121 solution, in which the PBS concentration is 1/5 to 1/2 of the normal concentration, while maintaining the dissolved state of the solution, and (iii) lyophilizing the obtained solution,
which is **characterized in that**, when the lyophilized product is dissolved in water for injection in a volume of 1/5 to 1/2 of the solution before (iii) lyophilization step prior to administration, the solution remains in solution state, in which the concentration of KUS121 is 0.100 to 0.625 mg/mL, and the concentration of PBS is the normal PBS concentration.

9. The lyophilized drug product of any one of claims 1 to **8,** wherein the KUS solution comprising a PBS buffer before (iii) lyophilization step is dispensed into each vial in which the amount of KUS121 is 20 to 125 µg per vial, said drug product is stored in the vial in lyophilized state.

10. A vial containing a lyophilized drug product comprising KUS121 which is for vitreous injection, wherein the lyophilized drug product comprises 20 to 125 µg of KUS121 and PBS buffer dry components corresponding to 0.1 to 0.4 mL of a normal PBS buffer solution, excluding water.

11. A method for preparing a lyophilized drug product that can be dissolved in water for injection to prepare a PBS buffer solution comprising 0.100 to 0.625 mg/mL of KUS121, wherein the PBS concentration is the normal PBS concentration, comprising
dissolving KUS121 in water for injection or a PBS buffer solution more dilute than usual,
adding a PBS buffer thicker than usual to the solution while maintaining the dissolved state of the solution, and
lyophilizing the obtained solution.

12. The method of claim 11, wherein the normal PBS concentration is a concentration of a PBS buffer with an osmotic pressure of 230 to 360 mOsm/kg, and a pH of 6.8 to 8.2.

13. The method of claim 11 or 12, wherein the PBS buffer thicker than usual has a concentration 2 to 10 times higher than the normal PBS concentration.

14. The method of any one of claims 11 to 13, wherein the PBS concentration of the solution before (iii) lyophilization step is 1/5 to 1/1 of the normal PBS concentration.

15. The method of any one of claims 11 to 14, wherein the volume of the water for injection added to the lyophilized product is 1/5 to 1/1 of the volume of the solution before (iii) lyophilization step.

16. The method of any one of claims 11 to 14, wherein the PBS buffer solution comprising KUS121 can be prepared by dissolving the lyophilized drug product in 200 µL of water for injection per vial.

17. The method of any one of claims 11 to 16, wherein the concentration of KUS121 in the solution before the lyophilization step is 0.020 to 0.125 mg/mL.

18. The lyophilized drug product of any one of claims 1 to 9, wherein [Na⁺], [K⁺], [Cl⁻], and [PO₄³⁻] in a solution obtained by dissolving the lyophilized drug product in water for injection are 100 to 200 mmol/L, 0 to 6.5 mmol/L, 90 to 180 mmol/L, and 6 to 14 mmol/L, respectively.

19. The vial of claim 10, wherein [Na⁺], [K⁺], [Cl⁻], and [PO₄³⁻] in a solution obtained by dissolving the lyophilized drug product in water for injection are 100 to 200 mmol/L, 0 to 6.5 mmol/L, 90 to 180 mmol/L, and 6 to 14 mmol/L, respectively.

20. The method of any one of claims 11 to 17, wherein [Na⁺], [K⁺], [Cl⁻], and [PO₄³⁻] in a solution obtained by dissolving the lyophilized drug product in water for injection are 100 to 200 mmol/L, 0 to 6.5 mmol/L, 90 to 180 mmol/L, and 6 to 14 mmol/L, respectively.
